# EUROPEAN PATENT SPECIFICATION

(11) **EP 2 282 642 B1**
(45) Date of publication and mention of the grant of the patent: **24.07.2013**
(21) Application number: 09737933.3
(22) Date of filing: 12.03.2009
(51) Int. Cl.: A23F 5/02, A61K 36/74

(54) **PRODUCTS COMPRISING, AND USES OF, DECARBOXYLATED PHENOLIC ACIDS DERIVED FROM CHLOROGENIC ACIDS OF COFFEE**
PRODUKTE MIT AUS KAFFEE-CHLOROGENSÄUREN ABGELEITETEN DECARBOXYLIERTEN PHENOLSÄUREN UND DEREN VERWENDUNG
PRODUITS CONTENANT DES ACIDES PHÉNOLIQUES DÉCARBOXYLÉS DÉRIVÉS DES ACIDES CHLOROGÉNIQUES DU CAFÉ, ET UTILISATIONS ASSOCIÉES

(30) Priority: 30.04.2008 EP 08155434
(43) Date of publication of application: 16.02.2011
(73) Proprietor: Nestec S.A., 1800 Vevey (CH)
(72) Inventor: BEL-RHLID, Rachid, CH-1073 Savigny (CH); KRAEHENBUEHL, Karin, CH-1926 Fully (CH); CAVIN, Christophe, CH-1820 Montreux (CH); RAAB, Thomas Wolfgang, CH-1091 Grandvaux (CH); PAGE, Nicolas, CH-1010 Lausanne (CH)
(74) Representative: Lomholt, Stig Bredsted
(86) International application number: PCT/EP2009/052939
(87) International publication number: WO 2009/132889

(56) References cited:
- EP-A- 0 444 899
- EP-A- 1 172 112
- EP-A- 1 424 076
- EP-A- 1 674 106
- EP-A- 1 712 137
- WO-A-00/47045
- WO-A-97/22341
- WO-A-02/085397
- WO-A-2004/056733
- WO-A-2005/016018
- US-A1- 2007 224 668
- DATABASE WPI Week 200634 Thomson Scientific, London, GB; AN 2006-323355 XP002498373 & JP 2006 020526 A (KAWASAKI K) 26 January 2006 (2006-01-26)
- DATABASE WPI Week 200822 Thomson Scientific, London, GB; AN 2008-D03339 XP002536808 & CN 101 057 678 A (BIOVALLEY TECHNOLOGIES SHENZHEN CO LTD) 24 October 2007 (2007-10-24)

## Description

### Field of the invention

The present invention relates to uses of decarboxylated phenolic acid derived from chlorogenic acid of coffee as well as products comprising decarboxylated phenolic acid derived from chlorogenic acid of coffee, especially a coffee extract, and methods of producing such products.

### Background

Coffee and coffee active compounds such as caffeine and diterpenes (e.g. cafestol, kahweol) have been shown to induce detoxifying enzymes (e.g. glutathione-S-transferases, GST) (Cavin C. et al, 1998. The coffee-specific diterpenes cafestol and kahweol protect against aflatoxin B1-induced genotoxicity trough a dual mechanism. Carcinogenesis 19, 1369-1375; Cavin, C. et al, 2003. Coffee diterpenes prevent benzo[a]pyrene genotoxicity in rat and human culture systems. Biochemical Biophysical Research Communication 306, 488-495; Huber, W. et al. 2002a. Enhancement of the chemoprotective enzymes glucuronosyl transferase and glutathione transferase in specific organs of the rat by the coffee components kahweol and cafestol. Archive of Toxicology 76, 209-217). Increased GST activity by coffee has been further demonstrated in human following consumption of 800 ml of coffee for 5 days (Steinkellner, H. et al. 2005. Coffee consumption induces GSTP in plasma and protects lymphocytes against (+/-)-anti-benzo[a]pyrene-7,8-dihydrodiol-9,10-epoxide induced DNA-damage: results of controlled human intervention trials. Mut. Res. 591 264-275).

This kind of antioxidant activity is known to protect against "oxidative stress" by reducing damaging free radicals that may be implicated e.g. in cancer, heart disease, degenerative brain disorders and ageing.

Alzheimer's disease (AD) is a progressive neurodegenerative disease and the most common form of dementia, symptoms being e.g. memory loss, confusion, mood swings, and cognitive decline. It is characterized by the presence of extracellular amyloid plaques and intraneuronal neurofibrillary tangles in the brain, of which the main constituent is fibrillar aggregates of a 39-42 residue peptide referred to as the amyloid beta protein (Aβ). Aβ fibril formation is thought to play a central role in the etiology of AD. Several pathogenic AD mutations have been shown to result in increased Aβ levels, especially of the variant Aβ42. Amyloid fibril formation is therefore thought to be the cause of disease progression and neurodegeneration in AD. It has been demonstrated by in vitro studies that Aβ fibril formation occurs via a complex multistep mechanism that involves discrete soluble oligomeric intermediates termed ADDLS or protofibrils (PFs), which disappear upon fibril formation. This suggests that PFs may be AD's pathogenic species. A number of other diseases in humans and animals involve protein aggregation, e.g. macular degeneration, Bovine spongiform encephalopathy (BSE), Creutzfeldt-Jakob disease, and diabetes.

WO 02/085397 a raw green coffee bean extraction method and coffee extract end product. The method produces a coffee extract end product which contains phenolic compounds.

JP-A-2006/020526 discloses a coffee flavor composition having improving aroma and taste. Further JP-A-2006/020526 discloses a roast coffee extract said to have an antioxidant effect.

WO-A-00/4704 discloses the use of 4-VG as an antioxidant and as a functional flavor. It is not concerned with coffee extracts.

WO-A-2004/05673 discloses a microwave induced process for the preparation of substituted 4-vinylphenols in which perfumery and flavouring vinylphenols.

US-A-2007/0224668 discloses 4-vinylguaiacol produced using recombinant E. coli containing a decarboxylase gene from Bacillus pumilis.

EP 1674 196 discloses a bone remodeling composition containing methoxphenyl compounds.

To increase the health benefits of food and beverage products there is a desire to produce products with an increased antioxidant activity, as well as other beneficial biological activities, and to find natural sources of antioxidants and other compounds with beneficial biological activities, that can be used to enhance the properties of food and beverage products as well as e.g. in cosmetic and medical products.

### Summary of the invention

The inventors have now found that decarboxylated phenolic acids derived from chlorogenic acid of coffee have antioxidant and anti-inflammatory properties, as well as being effective to inhibit and/or retard the aggregation of amyloid beta peptides, and that they can be produced from a coffee extract, yielding a coffee extract with enhanced antioxidant and anti-inflammatory properties. Accordingly, the invention relates to a method of producing a coffee extract comprising a compound selected among 4-vinylcatechol, 4-vinylguaiacol, 4-vinylveratrole, and mixtures thereof, the method comprising: a) extracting coffee beans with water and/or steam to produce a coffee extract; and b) treating the coffee extract to hydrolyse chlorogenic acid present in the extract to phenolic acid, and to decarboxylate the resulting phenolic acid. In further aspects the invention relates to a a coffee extract comprising a compound selected among 4-vinylcatechol, 4-vinylguaiacol, 4-vinylveratrole, and mixtures thereof, as described in claim 5; a method of producing a food or beverage product as described in claim 7; and to uses of a compound selected among 4-vinylcatechol, 4-vinylguaiacol, 4-vinylveratrole, and mixtures thereof

### Brief description of the figures

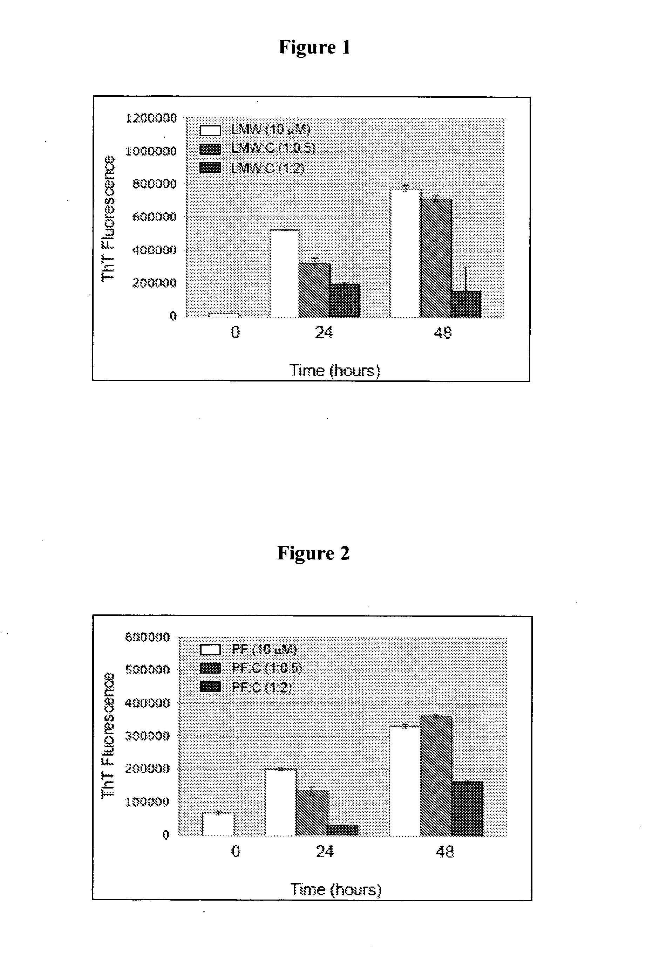

Figure 1 shows the results of an assay of the ability of 4-vinylcatechol to reduce and/or block the formation of amyloid fibrils from monomeric amyloid beta peptides. White bars are control; light grey bars are a ratio of Aβ42 to 4-vinylcatechol of 1:0.5 (molar ratio); dark grey bars are at a ratio of Aβ42 to 4-vinylcatechol of 1:2 (molar ratio).
Figure 2 shows the results of an assay of the ability of 4-vinylcatechol to reduce and/or block the formation of amyloid fibrils from protofibrils of amyloid beta peptides. White bars are control; light grey bars are a ratio of Aβ42 to 4-vinylcatechol of 1:0.5 (molar ratio); dark grey bars are at a ratio of Aβ42 to 4-vinylcatechol of 1:2 (molar ratio).

### Detailed description of the invention

Chlorogenic acids are a family of esters formed between trans-cinnamic acids and quinic acid. Chlorogenic acids are naturally present in coffee, mainly as mono- and di-esters of quinic acid and phenolic groups (e.g. caffeic, ferulic, coumaric, methoxycinnamic) attached to different positions. Chlorogenic acids may be hydrolysed to yield phenolic compounds such as caffeic acid and ferulic acid. These phenolic compounds can be further transformed by decarboxylation. This invention relates to decarboxylated phenolic acid derived from chlorogenic acid of coffee. By the term chlorogenic acid of coffee is meant one or more chlorogenic acids that are naturally found in coffee and that contain a phenolic group, whether actually derived from coffee or from another source. In a preferred embodiment chlorogenic acid of coffee is actually derived from coffee. Chlorogenic acids naturally present in coffee are e.g. caffeoyl quinic acids (CQA) (such as e.g. 3-, 4-, or 5-caffeoyl quinic acid), and diesters, feruloyl quinic acids (FQA) (such as e.g. 3-, 4-, or 5-feruloyl quinic acid) and diesters, and dimethoxycinnamoyl quinic acids (DMCQA) (such as e.g. 3-, 4-, or 5-dimethoxycinnamoyl quinic acid) and diesters.

Chlorogenic acids of coffee may be hydrolysed to generate phenolic acids, e.g. CQA may be hydrolysed to generate caffeic acid (CA), FQA may be hydrolysed to generate ferulic acid (FA), and DMCQA may be hydrolysed to generate dimethoxycinnamic acid (DMCA). Phenolic acids generated by the hydrolysis of chlorogenic acids of coffee may further be decarboxylated to generate decarboxylated phenolic acid derived from chlorogenic acid of coffee; e.g. CA may be decarboxylated to generate 4-vinylcatechol, FA may be decarboxylated to generate 4-vinylguaiacol, and DMCA may be decarboxylated to generate 4-vinylveratrole.

The decarboxylated phenolic acid derived from chlorogenic acid of coffee is selected among 4-vinylcatechol, 4-vinylguaiacol, 4-vinylveratrole, and mixtures thereof

The invention relates to a coffee extract comprising decarboxylated phenolic acid derived from chlorogenic acid of coffee. The coffee extract of the invention may be an extract of roasted coffee beans, green coffee beans, or both.

The coffee extract comprises at least 0.1 milligram total of 4-vinylcatechol, 4-vinylguaiacol and 4-vinylveratrole per gram of dry matter, such as at least 1 at least 2, at least 5 or at least 20 milligram per gram of dry matter. In another embodiment the coffee extract comprises at least 0.1 milligram of 4-vinylcatechol per gram of dry matter, such as at least 1 at least 2, at least 5 or at least 20 milligram per gram of dry matter.

According to the method of the invention, chlorogenic acids may be transformed into decarboxylated phenolic acid derived from chlorogenic acid of coffee by hydrolysing chlorogenic acid into phenolic acid and decarboxylating the resulting phenolic acid, as described above.

The hydrolysis and decarboxylation reactions may be performed separately or they may be overlapping in time.

The transformation of chlorogenic acids may be performed by any suitable method. In one embodiment of the invention the transformation is performed by one or more microorganisms capable of transforming chlorogenic acids in the coffee. Microorganisms capable of transforming chlorogenic acids may e.g. be identified as disclosed in the examples of this application. Suitable microorganisms may be yeast, e.g. Bakers yeast; fungi, e.g. an *Aspergillus;* or bacteria, e.g. a lactic acid bacteria, e.g. *a Lactobacillus,* such as e.g. *L. johnsonii* (CNCM I-1225). In one embodiment of the invention the microorganism capable of transforming chlorogenic acids is a lactic acid bacterium. In another embodiment of the invention two or more microorganisms are used to transform chlorogenic acids, e.g. one or more microorganisms capable of hydrolysing chlorogenic acids into phenolic acid, and one or more microorganisms capable of decarboxylating phenolic acid.

Transformation of chlorogenic acids may be performed by incubating the coffee extract with a microorganism capable of transforming chlorogenic acids under conditions suitable for the growth of the specific microorganism for the time necessary to achieve the required transformation of chlorogenic acids. The specific conditions can easily be determined by the skilled person, e.g. with reference to the examples contained herein.

In another embodiment of the invention the transformation of chlorogenic acids is performed by one or more suitable microorganisms by the use of non-replicative microorganisms (e.g. lysed microbial cells). By incubating coffee extract with lysed cells under suitable conditions, enzymes present in the cell lysate may transform chlorogenic acids. Suitable cells may e.g. be cells of the microorganisms mentioned above. Suitable methods for producing cell lysate are known in the art.

The amount of microorganism and conditions of the transformation should be suitable to achieve the desired transformation of chlorogenic acids, and can be determined by the skilled person by routine methods, e.g. using the methods disclosed in the examples herein.

In another embodiment the transformation of chlorogenic acids is performed by the use of one or more enzymes capable of transforming chlorogenic acids. In one embodiment at least two enzymes are used, at least one enzyme capable of hydrolysing chlorogenic acids to generate phenolic acid, and at least one enzyme capable of decarboxylating the resulting phenolic acid. A suitable enzyme for hydrolysing chlorogenic acid is e.g. an esterase e.g. chlorogenate esterase derived from *Aspergillus japonicus* (Kikkoman, Japan). A suitable enzyme for decarboxylating phenolic acids is e.g. a decarboxylase (EC 4.1.1.X), e.g. pyruvate decarboxylase (EC 4.1.1.1). The enzymatic transformation may be performed by conventional methods for enzymatic reactions, e.g. by dissolving or suspending enzyme(s) in the coffee extract under conditions suitable for the required enzyme activity. The enzyme(s) may be inactivated, e.g. by heating, after the transformation has taken place. Enzyme(s) to be used may also be immobilised, e.g. on a membrane or on an inert carrier, and the coffee extract to be treated may be circulated over the membrane or through the carrier until the desired degree of transformation has been achieved. When two or more enzymes are used, they may be used simultaneously, or the treatment may be performed sequentially, e.g. if the optimal conditions vary between the enzymes.

The amount of enzyme and the conditions to be used should be suitable to achieve the desired hydrolysis of chlorogenic acids and decarboxylation of phenolic acids, and can be determined by the skilled person by routine methods.

The coffee beans to be extracted to produce the coffee extract may be whole or ground. In one embodiment of the invention the coffee beans are green coffee beans. In a further embodiment green coffee beans are co-extracted with roasted coffee beans, i.e. green and roasted coffee beans are extracted simultaneously in the same extraction system to yield a mixed extract. The most volatile aroma components may be stripped from the beans before extraction, e.g. if the extract is to be used for the production of pure soluble coffee. Methods for stripping of volatile aroma components are well known in the art, e.g. from EP 1078576.

The coffee beans to be extracted may be extracted by any suitable method yielding an extract comprising chlorogenic acids. Extraction of coffee beans with water and/or steam is well known in the art, e.g. from EP 0916267. The extract may undergo a concentration step and may be dried before the treatment to transform chlorogenic acids, e.g. by spray drying or freeze drying. If the extract has been dried it may be resuspended if required to effect the treatment to transform chlorogenic acids. The coffee extract may undergo any suitable treatment to remove undesired components of the extract before, during or after the transformation of chlorogenic acids, e.g. to increase the concentration of decarboxylated phenolic acid in the final treated extract.

The treatment of the extract to transform chlorogenic acids may be performed after or during the extraction. The extract may be separated from the extracted coffee beans before, during or after the treatment to transform chlorogenic acids. In one embodiment the extract is kept separate from the extracted coffee beans after the treatment to transform chlorogenic acids, i.e. the extract is not brought into contact with the extracted coffee beans again after the treatment to transform chlorogenic acids. Separation of the extract from the extracted coffee beans may be performed by any suitable method, e.g. filtration or centrifugation. The separation may be performed to the extent practically and economically feasible and needed in view of the desired use of the extract. The separation may thus not be 100% complete, e.g. a minor part of undissolved material from the beans may still be present with the extract after separation.

The invention also relates to a method of producing a food or beverage product wherein a coffee extract of the invention is used as an ingredient of said food or beverage product. In one embodiment of the invention the extract is used separately from the extracted coffee beans, i.e. undissolved material from the beans is substantially removed by separation as described herein and is not used in the production of the food or beverage product. The food or beverage product may be any food or beverage product known in the art. In a preferred embodiment the food or beverage product is a coffee beverage; pure soluble coffee; a soft drink; a dietary supplement; a dairy product; a cereal product; a fruit or vegetable juice product; or a confectionery product, such as a chocolate product, e.g. a chocolate drink. A soluble coffee product may be produced by concentrating and drying the extract of the invention. Before drying, the extract may be mixed with coffee extract that has not been treated to transform chlorogenic acids, e.g. extract of roasted coffee beans, green coffee beans, or both. Methods for producing a soluble coffee product from a coffee extract are well known in the art. When the extract is used for the production of a coffee product, the beans to be extracted may have been subjected to stripping to remove volatile aromas before extraction, e.g. as described in EP-A-1078576. The volatile aromas may then be added back to the extract after the treatment to hydrolyse chlorogenic acids, e.g. after drying, to produce an aromatised soluble coffee product. A soluble coffee product produced from a coffee extract of the invention may be sold as such, or may e.g. be mixed with a creamer and/or sweetener and sold to prepare a coffee beverage comprising creamer and/or sweetener, e.g. cappuccino or café latte.

Also disclosed is a food or beverage product comprising at least 0.1 mg of 4-vinylcatechol and/or methoxy derivative thereof per gram of dry matter; such as at least 1 at least 2, at least 5 or at least 20 milligram per gram of dry matter.

When a coffee extract according to the invention is used as an ingredient of a food or beverage product it may be added at any appropriate step in the production process of said food or beverage product to achieve the desired effect. The extract may be added in any amount suitable to bring about the desired effect, e.g. antioxidant effect or anti-inflammatory effect.

Also disclosed herein is a food or beverage product comprising decarboxylated phenolic acid derived from chlorogenic acid of coffee. The food or beverage product may be any food or beverage product known in the art. In a preferred embodiment the food or beverage product is a coffee beverage; pure soluble coffee; a soft drink; a dietary supplement; a dairy product; a cereal product; a fruit or vegetable juice product; or a confectionery product, such as a chocolate product, e.g. a chocolate drink. The food or beverage product may e.g. be prepared by the methods disclosed herein.

### Use of decarboxylated phenolic acid derived from chlorogenic acid of coffee

The invention also relates to use of a compound selected among 4-vinylcatechol, 4-vinylguaiacol, 4-vinylveratrole, and mixtures thereof, as described in claims 9-13. Decarboxylated phenolic acids to be used according to the invention may be produced by any suitable method, e.g. by decarboxylation of caffeic acid and may be in any suitable form, e.g. as purified compound(s). In one embodiment of the invention decarboxylated phenolic acid to be used according to the invention is in the form of a coffee extract comprising decarboxylated phenolic acid as disclosed herein. In another embodiment of the invention decarboxylated phenolic acid is partly or completely isolated from a coffee extract of the invention. Decarboxylated phenolic acid to be used according to the invention may be administered by any suitable method to a human or animal, e.g. orally, intravenously, or topically to the skin. If administered orally this may e.g. be in the form of a food or beverage product of the invention. In one embodiment of the invention a food or beverage product of the invention is sold with labelling indicating a use according to the invention.

Disclosed herein is the use of decarboxylated phenolic acid derived from chlorogenic acid of coffee for preparing a food or beverage product. The food or beverage product may be any food or beverage product known in the art. In a preferred embodiment the food or beverage product is a coffee beverage; pure soluble coffee; a soft drink; a dietary supplement; a dairy product; a cereal product; a fruit or vegetable juice product; or a confectionery product, such as a chocolate product, e.g. a chocolate drink. When decarboxylated phenolic acid is used to prepare a food or beverage product it may be added at any appropriate step in the production process of said food or beverage product.

Disclosed herein is the use of a compound selected among 4-vinylcatechol, 4-vinylguaiacol, 4-vinylveratrole, and mixtures thereof as an antioxidant, e.g. as an ingredient in a product, e.g. a food or beverage product, wherein antioxidant properties are desired, e.g. to prevent oxidation of components of the product during storage. Antioxidants are commonly used in a number of products and a compound selected among 4-vinylcatechol, 4-vinylguaiacol, 4-vinylveratrole, and mixtures thereof may be used in a similar way as conventional antioxidants. The skilled person can easily determine the amount needed to achieve the desired antioxidant effect by routine experimentation.

In one embodiment the invention relates to use of a compound selected among 4-vinylcatechol, 4-vinylguaiacol, 4-vinylveratrole, and mixtures thereof to enhance antioxidant and/or anti-inflammatory capacity *in vivo* in a human or animal, e.g. by inducing detoxifying enzymes such as gluthathione-S-transferase (GST) and by increasing the Nrf2-mediated gene expression pathway. Increased Nrf2 activity associated genes have been reported to enhance detoxification and to stimulate the endogenous defence against oxidative stress. These effects may e.g. be achieved by oral administration of the decarboxylated phenolic acids or by topical application to the skin of a human or an animal.

In a further embodiment the invention relates to use of a compound selected among 4-vinylcatechol, 4-vinylguaiacol, 4-vinylveratrole, and mixtures thereof to decrease inflammation in a human or animal, e.g. by reducing the prostaglandin E2 level, e.g. by orally administering a compound selected among 4-vinylcatechol, 4-vinylguaiacol, 4-vinylveratrole, and mixtures thereof to a human or animal.

Many health problems and disorders are related to oxidative stress and inflammation. A compound selected among 4-vinylcatechol, 4-vinylguaiacol, 4-vinylveratrole, and mixtures thereof may be used to treat or prevent such problems or disorders. Relevant problems and disorder are e.g. skin disorders, e.g. photodamage caused by UV-radiation, atopic dermatitis, eczema, scaling, itching, allergic symptoms; brain disorders; inflammation; obesity; and cancer, e.g. skin cancer and lung cancer.

In one embodiment of the invention a compound selected among 4-vinylcatechol, 4-vinylguaiacol, 4-vinylveratrole, and mixtures thereof is used as an anti-diabetic agent, e.g. by reducing blood glucose levels, and/or increasing blood levels of leptin, insulin and/or c-peptide; as a bone remodelling agent, e.g. by increasing bone mineral density and/or by increasing serum levels of estrogen and/or progesterone and/or alkaline phosphataise activity; as anti-metastactic agents, e.g. with anti-angiogenic effect; and/or for brain protection. These effects may e.g. be achieved by oral administration to a human or an animal.

In a further embodiment of the invention a compound selected among 4-vinylcatechol, 4-vinylguaiacol, 4-vinylveratrole, and mixtures thereof is used for the preparation of a formulation to treat or prevent, diabetes, brain disorders, inflammation, obesity, cancer; neurodegenerative disorders, cognitive decline, mild cognitive impairment, dementia, mood disorders, depression, sleep disorders, a disease involving protein aggregation, Alzheimer's disease (including common symptoms of AD, dementia, mild cognitive impairment and cognitive decline like sleep disorders, mood swings, depression, stress), macular degeneration, or diabetes. The formulation may be in any suitable form, e.g. for oral administration or topical administration to the skin, e.g. in the form of a food or beverage product, a nutritional supplement, a tablet, a lotion, or a cosmetic product. In a preferred embodiment the formulation is a medicament.

The invention further relates to non-therapeutical use of a food product, beverage product, food supplement or pet food product of the invention, for treating and/or preventing skin disorders, e.g. photodamage caused by UV-radiation, atopic dermatitis, eczema, scaling, itching, allergic symptoms; inflammation; obesity; cancer, e.g. skin cancer and lung cancer; cognitive decline, mood disorders, and/or sleep problems; for brain protection; and/or for improving cognitive performance, immune response, and/or gut barrier function in a human or animal. Cognitive performance may e.g. be expressed as ability and speed of learning, ability and speed of solving intellectual problems, ability to form and recall memories, reaction time, and the like. Cognitive decline may e.g. manifest itself as reduced memory, forgetfulness, word or name-finding problems, decline in memory, concentration, ability to plan or organise, ability to perform complex tasks, and/or cognitive performance, and may e.g. result from age, stress, disease, or other grounds. Cognition is understood as mental processes such as comprehension, inference, decision-making, planning, learning, memory, association, concept formation, language, attention, perception, action, problem solving and mental images.

In a further disclosure, methods of improving cognitive performance; treating or preventing skin disorders, e.g. photodamage caused by UV-radiation, atopic dermatitis, eczema, scaling, itching, allergic symptoms; inflammation; obesity; cancer, e.g. skin cancer and lung cancer; neurodegenerative disorders; cognitive decline; mild cognitive impairment; dementia; a disease involving porotein aggregation; Alzheimer's disease; macular degeneration; or diabetes are disclosed; the method comprising administering a food product, beverage product or pet food product comprising an effective amount of a compound selected among 4-vinylcatechol, 4-vinylguaiacol, 4-vinylveratrole, and mixtures thereof, to a human or animal. The food product, beverage product or pet food product may be administered concomitantly with a medicament to increase the efficacy and/or reduce the dose of the medicament.

In a still further disclosure, a method of treating or preventing skin disorders, e.g. photodamage caused by UV-radiation, atopic dermatitis, eczema, scaling, itching, allergic symptoms; inflammation; obesity; cancer, e.g. skin cancer and lung cancer; neurodegenerative disorders; cognitive decline; mild cognitive impairment; dementia; a disease involving protein aggregation; Alzheimer's disease; macular degeneration; or diabetes is described; comprising administering an effective amount of a medicament comprising a compound selected among 4-vinylcatechol, 4-vinylguaiacol, 4-vinylveratrole, and mixtures thereof to a human or animal in need thereof. The food product, beverage product or pet food product may be administered concomitantly with a medicament to increase the efficacy and/or reduce the dose of the medicament.

### EXAMPLES

### Example 1

### Treatment of green coffee extract with a spray-dried preparation of Lactobacillus johnsonii (CNCM I-1225)

30 mg of a dried green coffee extract was dissolved in 1 ml phosphate buffer (50 mM, pH 7.0) or in 1 ml water. To this solution, 10 mg of a spray-dried preparation of *Lactobacillus johnsonii* (CNCM 1-1225) (3.3 E9 cfu/g) was added. The mixture was then incubated at 37°C and samples were withdrawn at different reaction times. After centrifugation (3000 g, 5 min) and filtration (0.45 µm pore size syringe filters, Millipore SLHA 025 BS) the samples were analysed by HPLC.

### HPLC analysis

Coffee extract samples were diluted to 1% w/w and analyzed by RP-HPLC on a CC 250/4 Nucleosil 100-5-C18 column (Macherey-Nagel). The eluent system was Millipore water, 0.1% TFA and CH₃CN at a flow rate of 1 mL/min. The method allowed the simultaneous determination of CQA's, FQA's, di-CQA's, caffeic acid (CA), ferulic acid (FA), and 4-vinylcatechol (absorbance at 325 nm) using external standard calibration curves. Results were expressed relative to the reference at time 0 (t0).

### Antioxidant Responsive Element (ARE) luciferase assay

The pGL-8xARE which contains eight copies of the ARE present in rat glutathione-S-transferase A2 (GSTA2) along with the pcDNA3.1 plasmid containing the neomycin selectable marker was stably transfected into human MCF7 cells (Wang et al., Cancer Res. 66, 10983-10994, 2006). ARE (antioxidant-responsive element) is the binding site of the transcription factor Nrf2 which regulates the genes involved in detoxification and endogenous defence against oxidative stress. The plasmid pGL-8xARE contains a luciferase gene downstream of the eight Nrf2 binding sites that allows monitoring Nrf2 activity. The AREc 32 cells were seeded in 96-well microtiter plates in DMEM growth medium. After treatment with 4-vinylcatechol for 24 h firefly luciferase activity was determined.

### Prostaglandin E2 formation assay

Human colon HT-29 cells were treated with 4-vinylcatechol for 15h followed by a co-incubation of 6 h together with a pro-inflammatory agent TNF-α (10 ng/ml). Analysis of the PGE2 production in HT-29 cells was determined using a competitive enzyme immunoassay (EIA) (Cavin et al., BBRC 327, 742-49, 2005).

### Results

An unknown peak was observed in HPLC analysis of fermented coffee extracts produced by the fermentation or incubation of 2 different extracts of green Robusta beans. The compound was identified by a combination ofLC-MS-ToF and NMR as 4-vinylcatechol. Green coffee extract was treated with as described above and analysed by HPLC. The results are shown in table 1.

**Table 1. Composition of green coffee extract treated with Lactobacillus johnsonii for specified time. Amounts given in % of the amounts in the untreated extract except for 4-vinylcatechol which is given as the area of the HPLC-signal. 4-vinylcatechol was not detected in untreated extract.**

| time (h) | 16 | 24 |
|---|---|---|
| CQA | 38 | 32 |
| FQA | 45 | 42 |
| diCQA | 27 | 20 |
| CA | 7409 | 549 |
| FA | 847 | 422 |
| 4-vinylcatechol (area of HPLC peak, AU) | 643 | 2119 |

Induction of Nrf2 activity by 4-vinylcatechol is shown in table 2.

**Table 2. Induction of Nrf2 activity by 4-vinylcatechol (firefly luciferase activity, AU).**

| 4-vinylcatechol (µg/ml) | firefly luciferase activity (AU) |
|---|---|
| 200 | 16 |
| 400 | 41 |
| 600 | 22 |

PGE2 production in HT-29 cells is shown in Table 3.

**Table 3. PGE2 production in HT-29 cells relative to untreated control sample**

| 4-vinylcatechol (µg/ml) | PGE2 formation (% of control) |
|---|---|
| 0 | 100 |
| 3.13 | 58 |
| 6.25 | 46 |
| 9.38 | 21 |
| 15.63 | 12 |
| 31.25 | 5 |
| 62.5 | 2 |

### Example 2

Green coffee extract was diluted to 1% w/w and analyzed by RP-HPLC on a CC 250/4 Nucleosil 100-5-C18 column (Macherey-Nagel). The eluent system was Millipore water, 0.1% TFA and CH₃CN at a flow rate of 1 mL/min. 4-vinylcatechol was detected by absorbance at 265 nm. A standard calibration curve was obtained for 4-vinylcatechol by external calibration with 4-vinylguaicol, as 4-vinylcatechol is unstable in isolated form. Results are shown in table 4.

**Table 4. Composition of green coffee extract treated with Lactobacillus johnsonii for specified time. Amounts given in milligram per gram of dry coffee extract. 4-vinylcatechol was not detected in untreated extract.**

| | | |
|---|---|---|
| time (h) | 16 | 24 |
| 4-vinylcatechol | 11 | 35 |

### Example 3

### Monomeric Aβ

Monomeric Aβ42 peptides were purified by size exclusion chromatography and incubated at 37°C at a concentration of 10 µM with 4-vinylcatechol at a ratio of Aβ42 to the tested compound of 1:0.5 and 1:2 (molar ratio). The extent of aggregation was assessed at 24 and 48 hours by Thioflavin T (ThT) fluorescence. Controls were performed in the same way except for the absence of a compound to be tested. ThT is a hydrophobic dye that exhibits enhanced fluorescence upon binding to amyloid fibrils. ThT binds specifically to amyloid fibrils, but not monomeric forms of Aβ. In this assay a decrease or absence of ThT fluorescence indicated that the molecule being tested reduced and/or blocked the formation of amyloid fibrils. The results of this assay are shown in fig. 1.

### Protofibrillar Aβ

Size exclusion purified protofibrillar mixture of Aβ42 was incubated at 37°C at a concentration of 10 µM with 4-vinylcatechol at a ratio of Aβ42 to the tested compound of 1:0.5 and 1:2 (molar ratio). The extent of aggregation was assessed at 24 and 48 hours by Thioflavin T (ThT) fluorescence. Controls were performed in the same way except for the absence of a compound to be tested. A decrease or absence of an increase in ThT fluorescence signal of protofibrils indicated that the molecule being tested reduced and/or blocked the formation of amyloid fibrils. The results of this assay are shown in fig. 2.

## Claims

1. A method of producing a coffee extract comprising a compound selected among 4-vinylcatechol, 4-vinylguaiacol, 4-vinylveratrole, and mixtures thereof; the method comprising:
a) extracting coffee beans with water and/or steam to produce a coffee extract; and
b) treating the coffee extract to hydrolyse chlorogenic acid present in the extract to phenolic acid, and to decarboxylate the resulting phenolic acid.

2. The method of claim 1 wherein the hydrolysis of chlorogenic acids and the decarboxylation of phenolic acid in step b) is performed by a microorganism.

3. The method of claim 2 wherein the microorganism is a lactic acid bacterium.

4. The method of any of claims 1-3 wherein the coffee beans to be extracted are green coffee beans.

5. A coffee extract comprising at least 0.1 milligram total of 4-vinylcatechol, 4-vinylguaiacol and 4-vinylveratrole per gram of dry matter.

6. The coffee extract of claim 5 6 being an extract of green coffee beans

7. A method of producing a food or beverage product wherein a coffee extract according to any of claims 5-6 is used as an ingredient of said food or beverage product.

8. The method of claim 8 wherein the food or beverage product is a coffee beverage, pure soluble coffee, a soft drink, a dietary supplement, a dairy product, a cereal product, a fruit or vegetable juice product, or a confectionery product.

9. Use of a compound selected among 4-vinylcatechol, 4-vinylguaiacol, 4-vinylveratrole, and mixtures thereof for the preparation of a medicament.

10. Use of a compound selected among 4-vinylcatechol, 4-vinylguaiacol, 4-vinylveratrole, and mixtures thereof for the preparation of a formulation to treat or prevent brain disorders, inflammation, obesity, cancer, neurodegenerative disorders, cognitive decline, mild cognitive impairment, dementia, mood disorders, depression, sleep disorders, a disease involving protein aggregation, Alzheimer's disease, macular degeneration, or diabetes.

11. The use of claim 11 wherein said formulation is a food or beverage product.

12. Use of a compound selected among 4-vinylcatechol, 4-vinylguaiacol, 4-vinylveratrole, and mixtures thereof to promote bone remodelling.

13. Use of a compound selected among 4-vinylcatechol, 4-vinylguaiacol, 4-vinylveratrole, and mixtures thereof to increase the antioxidant capacity *in vivo* of a human or an animal and/or for brain protection.

## Patentansprüche

1. Verfahren zur Herstellung eines Kaffeeextraktes, das eine Verbindung aufweist, die aus 4-Vinylcatechol, 4-Vinylguajacol, 4-Vinylveratrol und Mischungen daraus ausgewählt ist, wobei das Verfahren folgendes aufweist:
a) Extrahieren von Kaffeebohnen mit Wasser und/oder Dampf, um einen Kaffeeextrakt herzustellen, und
b) Behandeln des Kaffeeextraktes, um in dem Extrakt vorhandene Chlorogensäure zu Phenolsäure zu hydrolysieren, und die resultierende Phenolsäure zu decarboxylieren.

2. Verfahren nach Anspruch 1, wobei die Hydrolyse von Chlorogensäuren und die Decarboxylierung von Phenolsäure in Schritt b) durch einen Mikroorganismus durchgeführt werden.

3. Verfahren nach Anspruch 2, wobei der Mikroorganismus ein Milchsäurebakterium ist.

4. Verfahren nach einem der Ansprüche 1 bis 3, wobei die zu extrahierenden Kaffeebohnen grüne Kaffeebohnen sind.

5. Kaffeeextrakt mit mindestens insgesamt 0,1 Milligramm 4-Vinylcatechol, 4-Vinylguajacol und 4-Vinylveratrol pro Gramm Trockenmasse.

6. Kaffeeextrakt nach Anspruch 5, wobei der Kaffeeextrakt ein Extrakt aus grünen Kaffeebohnen ist.

7. Verfahren zur Herstellung eines Nahrungsmittel- oder Getränkeproduktes, wobei ein Kaffeeextrakt nach einem der Ansprüche 5 bis 6 als Zutat des Nahrungsmittel- oder Getränkeprodukts verwendet wird.

8. Verfahren nach Anspruch 8, wobei das Nahrungsmittel- oder Getränkeprodukt ein Kaffeegetränk, reiner löslicher Kaffee, ein Erfrischungsgetränk, ein Nahrungsergänzungsmittel, ein Milchprodukt, ein Getreideprodukt, ein Obst- oder Gemüsesaftprodukt oder ein Süßwarenprodukt ist.

9. Verwendung einer Verbindung, die zur Herstellung eines Medikamentes aus 4-Vinylcatechol, 4-Vinylguajacol, 4-Vinylveratrol und Mischungen daraus ausgewählt ist.

10. Verwendung einer Verbindung, ausgewählt aus 4-Vinylcatechol, 4-Vinylguajacol, 4-Vinylveratrol und Mischungen daraus, zur Herstellung einer Formulierung zur Behandlung oder Vorbeugung von Gehirnerkrankungen, Entzündungen, Adipositas, Krebs, neurodegenerativen Erkrankungen, kognitiven Beeinträchtigungen, leichten kognitiven Störungen, Demenz, Gemütsstörungen, Depression, Schlafstörungen, einer Krankheit, die Proteinaggregation mit sich bringt, Alzheimer-Erkrankung, Makuladegeneration oder Diabetes.

11. Verwendung nach Anspruch 11, wobei die Formulierung ein Nahrungsmittel- oder Getränkeprodukt ist.

12. Verwendung einer Verbindung, die aus 4-Vinylcatechol, 4-Vinylguajacol, 4-Vinylveratrol und Mischungen daraus ausgewählt ist, um den Knochenumbau zu fördern.

13. Verwendung einer Verbindung, die aus 4-Vinylcatechol, 4-Vinylguajacol, 4-Vinylveratrol und Mischungen daraus ausgewählt ist, um die antioxidative Wirkung *in vivo* eines Menschen oder eines Tieres zu erhöhen, und/oder zum Schutz des Gehirns.

## Revendications

1. Procédé de production d'un extrait de café comprenant un composé sélectionné parmi le 4-vinylcatechol, le 4-vinylguaiacol, le 4-vinylveratrole et des mélanges de ces derniers ; le procédé comprenant :
a) l'extraction des grains de café avec de l'eau et/ou de la vapeur pour produire un extrait de café ; et
b) le traitement de l'extrait de café pour hydrolyser de l'acide chlorogénique présent dans l'extrait en acide phénolique, et pour décarboxyler l'acide phénolique résultant.

2. Le procédé selon la revendication 1 dans lequel l'hydrolyse des acides chlorogéniques et la décarboxylation de l'acide phénolique à l'étape b) sont réalisées par un micro-organisme.

3. Le procédé selon la revendication 2 dans lequel le micro-organisme est une bactérie d'acide lactique.

4. Le procédé de l'une des revendications 1-3 dans lequel les grains de café à extraire sont des grains de café vert.

5. Un extrait de café comprenant au moins un total de 0.1 milligramme de 4-vinylcatechol, de 4-vinylguaiacol et de 4-vinylveratrole par gramme de matière sèche.

6. L'extrait de café selon la revendication 5 étant un extrait de grains de café vert.

7. Procédé de production d'un produit alimentaire ou d'une boisson dans lequel un extrait de café selon l'une des revendications 5-6 est utilisé comme un ingrédient dudit produit alimentaire ou de ladite boisson.

8. Le procédé selon la revendication 8 dans lequel le produit alimentaire ou la boisson est une boisson au café, du café soluble pur, une boisson non alcoolisée, un complément alimentaire, un produit laitier, un produit céréalier, un produit de jus de fruit ou de légume, ou un produit de confiserie.

9. Utilisation d'un composé sélectionné parmi le 4-vinylcatechol, le 4-vinylguaiacol, le 4-vinylveratrole, et des mélanges de ces derniers pour la préparation d'un médicament.

10. Utilisation d'un composé sélectionné parmi le 4-vinylcatechol, le 4-vinylguaiacol, le 4-vinylveratrole, et des mélanges de ces derniers pour la préparation d'une formulation pour traiter ou prévenir les troubles cérébraux, l'inflammation, l'obésité, le cancer, les troubles neurodégénératifs, le déclin cognitif, la déficience cognitive légère, la démence, les troubles de l'humeur, la dépression, les troubles du sommeil, une maladie impliquant l'agrégation de protéine, la maladie d'Alzheimer, la dégénérescence maculaire, ou le diabète.

11. L'utilisation selon la revendication 11 dans laquelle ladite formulation est un produit alimentaire ou une boisson.

12. Utilisation d'un composé sélectionné parmi le 4-vinylcatechol, le 4-vinylguaiacol, le 4-vinylveratrole et des mélanges de ces derniers pour promouvoir le remodelage osseux.

13. Utilisation d'un composé sélectionné parmi le 4-vinylcatechol, le 4-vinylguaiacol, le 4-vinylveratrole et des mélanges de ces derniers pour augmenter la capacité antioxydante *in vivo* d'un humain ou d'un animal et/ou pour protéger le cerveau.
